# EUROPEAN PATENT SPECIFICATION

(11) **EP 2 654 723 B1**
(45) Date of publication and mention of the grant of the patent: **31.12.2014**
(21) Application number: 11813321.4
(22) Date of filing: 23.12.2011
(51) Int. Cl.: A61K 9/16, A61K 9/20

(54) **Solid pharmaceutical dosage forms comprising tadalafil and methods of preparation thereof**
Feste pharmazeutische Darreichungsformen enthaltend Tadalafil und deren Herstellungsverfahren
Formes pharmaceutiques solides comprenant du tadalafil et ses procédés de préparation

(30) Priority: 23.12.2010 WO PCT/EP2010/460052
(43) Date of publication of application: 30.10.2013
(73) Proprietor: Zaklady Farmaceutyczne Polpharma SA, 83-200 Starogard Gdanski (PL)
(72) Inventor: ZAKOWIECKI, Daniel, 83-200 Starogard Gdanski (PL); MAZGALSKI, Jaroslaw, 83-010 Straszyn (PL); TUKALSKA, Aleksandra, 83-200 Starogard Gdanski (PL)
(74) Representative: Tar, Miklos
(86) International application number: PCT/EP2011/006544
(87) International publication number: WO 2012/095151

(56) References cited:
- EP-A1- 1 985 310
- WO-A1-96/38131
- WO-A2-01/08688
- US-A1- 2010 099 687

## Description

The present invention relates to a process for preparing solid pharmaceutical composition containing tadalafil as active pharmaceutical ingredient. The invention furthermore relates to the pharmaceutical compositions containing tadalafil on the surface of a water-insoluble polymer.

### Background of the invention

Tadalafil is a drug used to treat male penile erectile dysfunction (impotence) and pulmonary arterial hypertension (ATC code: G04BE08). The drug works by selective inhibiting phosphodiesterase type 5 (PDE5) enzyme what cause relaxing muscles and increasing blood flow to particular areas of the organism.

Tadalafil is currently marketed under brand name Cialis by the Eli Lilly ICOS joint venture, and is used to treat erectile dysfunction in a men, although can also be used for other purposes. Cialis is available in different strengths, i.e. 5 mg, 10 mg, and 20 mg in the form of film-coated tablets for oral administration.

Tadalafil is β-carboline derivative, chemically described as (6*R-trans*)-6-(1,3-benzodioxol-5-yl)- 2,3,6,7,12,12a-hexahydro-2-methyl-pyrazino [1', 2':1,6] pyrido[3,4-*b*]indole-1,4-dione (CAS No. 171596-29-5). Its empirical molecular formula is C₂₂H₁₉N₃O₄, and its structural formula is given in Figure 1. Its molecular weight is 389.4 g/mol.

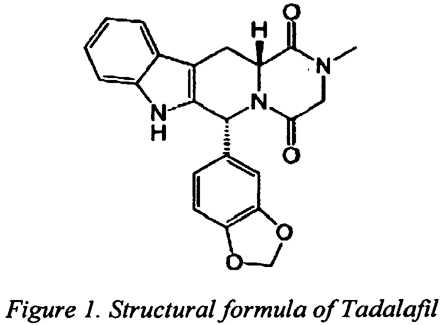

It is a well-known fact that the therapeutic effectiveness of a drug is closely related to its bioavailability, which depends on the solubility and permeability of the drug molecule. The low water solubility of a drug substance implies its low dissolution rate and consequently its low pharmaceutical availability.

Therefore improving the low solubility of the compounds which are insoluble or almost insoluble in water is one of problem to be solved to have a therapeutically effective formulation. Several solutions for improving the solubility were published in the literature. Such a solution can be the increased surface area for dissolution by decreasing the particle size by e.g. milling or micronization; or the use of solubility enhancing excipients like surfactants, cyclodextrins or waxy amphiphilic excipients in the formulation. Further solutions can be the use of more soluble salts, co-crystals, co-precipitates or prodrugs of the insoluble compound; or the use of special formulation techniques like the use of solid dispersions or nano-particles.

Tadalafil is a solid and is characterized as a BCS class IV compound because of its low solubility and low permeability. Its experimental water solubility is about 2 µg/mL at 37°C. Additionally it is characterized by low permeability which is a further limiting factor for pharmaceutical and biological availability of the drug.

In the international patent application No. WO 01/08688 A2 a pharmaceutical composition comprising the free drug particulate form of tadalafil and pharmaceutically acceptable salts and solvates thereof are disclosed. Said particles of the compound are characterized by particle size distribution, where at least 90% of particles have dimensions less than about 40 µm.

In the US Patent application No. 2010/0099687 A1 solid composites, preferably solid solutions comprising tadalafil and at least one carrier are disclosed, which are suitable for preparing pharmaceutical finished dosage forms. The active pharmaceutical ingredient and the carrier are "intimately associated" i.e. tadalafil is absorbed in the excipient forming a solid solution after the evaporation of the solvent, although no proof was disclosed to prove the existence of any interactions on molecular level.

In the US Patent No. 5,985,326 some improvement in the solubility of tadalafil was achieved by preparing formulations using co-precipitates of drug substance with a polymer as a carrier, like hydroxypropyl methylcellulose phtalate. Co-precipitate is obtained from aqueous medium in which said carrier is substantially insoluble.

It is also described in patent No. US 6,841,167 B1 pharmaceutical composition of tadalafil and pharmaceutically acceptable salts and solvates thereof, wherein a drug substance exist in form of free drug either dissolved or suspended in pharmaceutically acceptable solvent, and encapsulated in soft capsule shells as a solution or suspension.

It was unexpectedly found that extended dissolution rate of a pharmaceutical composition and pharmaceutical availability of tadalafil can be achieved by a specific fluid-bed granulation method resulting granulates having properties suitable for preparing pharmaceutical formulations.

### Brief description of the invention

The present invention relates to a process for preparing solid pharmaceutical composition containing tadalafil as active pharmaceutical ingredient by using fluid-bed hot-melt granulation process. The invention furthermore relates to the pharmaceutical compositions containing tadalafil on the surface of a water-insoluble polymer, namely ethylcellulose.

### Detailed description of the invention

The present invention provides an orally administered tadalafil drug formulation and the method of preparation thereof, which has improved pharmaceutical properties and biological availability.

In one embodiment, the present invention provides a process comprising the steps: a) preparing a solution of tadalafil in organic solvent; b) carrying up tadalafil onto a water-insoluble polymer ethylcellulose mixed with a diluent by fluid bed granulation; c) mixing the granulation with one or more further excipients.

In the preparation of solution of tadalafil the organic solvent can be a lower aliphatic alcohol like methanol, ethanol, isopropanol; or can be a keton like aceton or can be a mixture thereof. Preferably the solvent isopropanol or aceton, more preferably the mixture of aceton and isopropanol.

The use of active pharmaceutical ingredient (API) in crystalline or amorphous form can cause several problems during the technological process. Such a problems can be a unwanted change in physical properties like conversions between different polymorphic forms or a change in particle size, shape, surface properties. Using a solution of the API allows to avoid such limitations. Also, the possibility of eliminating such processes as water and other solvent adsorption and absorption by API, which translates to the creation of hydrates and solvates plays a significant role, together with the changes in melting temperature, solubility, hygroscopic parameters, mechanical properties of the substance and other parameters. Even minor changes in physical properties of the API can influence its bioavailability. Therefore according to the present invention API-in-solution is used as a starting material.

The granulation method also can play an important role in the formulation process and can have a significant influence on the properties of the composition. According to the present invention fluid-bed granulation method, preferably fluid-bed hot-melt granulation method is used at 50-150 °C. This method results a granulation of tadalafil having very advantageous properties for preparing solid pharmaceutical formulations. Surprisingly, if other granulation methods like wet granulation process is used the formulation prepared from the obtained granulation does not show these advantageous properties. In a comparative example it was found that the dissolution rate is significantly better in the case of the formulation which was prepared by using the granulation obtained by the fluid-bed hot-melt granulation method (Example 2, Figure 8.)

The other important factor is the polymer. The polymer used in the granulation step is water-insoluble polymer. It is a water-insoluble polymer which softens under high temperature used during this step. The water-insoluble polymer is ethylcellulose. The polymer is physiologically inert, tasteless and odorless hydrophobic polymer, mainly used to control or reduce the release rate of a soluble drug substance. But it was surprisingly found that the use of such polymer in combination with the fluid-bed hot-melt granulation method results a granulation in which the API is in a specific form. In this specific form tadalafil is not in crystalline or amorphous form but it is adsorbed on the surface of the water-insoluble polymer. From this granulation pharmaceutical composition can be formed with adding one or more pharmaceutically acceptable excipients. The compositions according to the present invention provide an improvement of dissolution rate of poorly soluble tadalafil, as is presented in the Figure 6.

The present invention is illustrated by the following examples.

A Glatt GPCG3 (Binzen, Germany) fluid bed apparatus was used for the granulation.

### Example 1.

Starting material, which is tadalafil in the mixture of acetone and isopropanol (API-in-solution) is combined with carrier (excipients) in the process of fluid hot melt granulation. Obtained blend is mixed with other excipients and finally with lubricant, then compressed to form tablets.

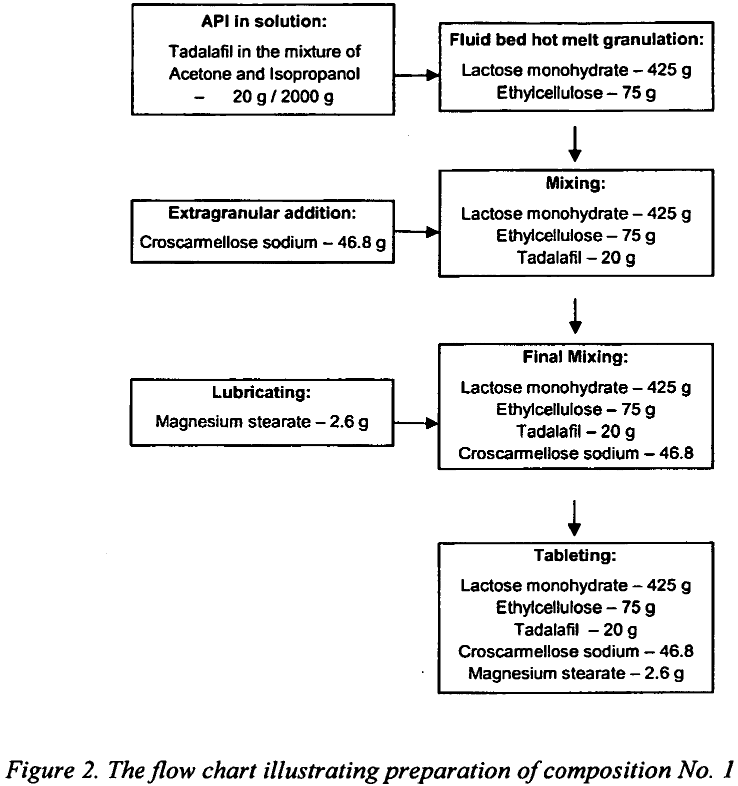

**Table 1. Fluid bed hot melt granulation, top spray, process parameters:**

| | |
|---|---|
| Inlet temperature (°C) | 80 C |
| Outlet temperature (°C) | 73 °C |
| Bed temperature (°C) | 71 °C |
| Fluidizing air flow (m³/h) | 85 m³/h |
| Coating rate (g/min) | 8 - 14 g/min |
| Atomizing air pressure (bar) | 0.5 bar |

**Table 2. Tadalafil tablets 10 mg - composition No. 1**

| Substance name | amount [mg/tabl] | amount [%] |
|---|---|---|
| Tadalafil | 10.0 | 3.51 |
| Lactose monohydrate | 212.5 | 74.64 |
| Ethylcellulose | 37.5 | 13.17 |
| Croscarmellose sodium | 23.4 | 8.22 |
| Magnesium stearate | 1.3 | 0.46 |
| Total: | 284.7 | 100.00 |

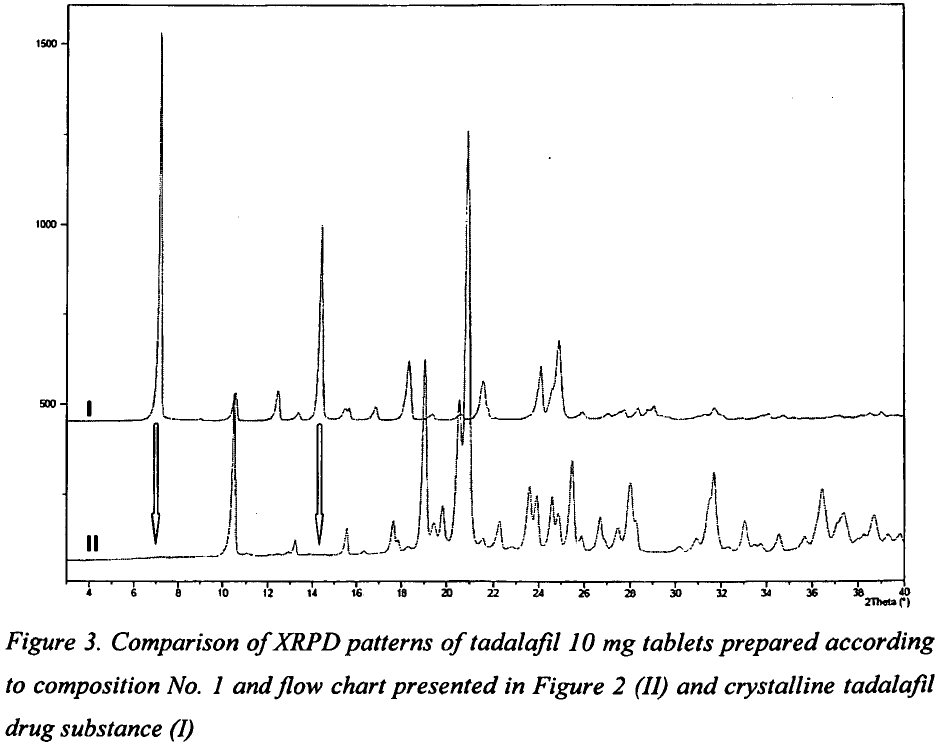

XRPD pattern of tadalafil 10 mg tablets prepared according to composition No. 1 (Table 2) and flow chart presented in Figure 2, shows lack of the most intensive peaks characteristic to crystalline tadalafil (marked with arrows). Moreover it is characterized by improved solubility of tadalafil, what is illustrated by means of dissolution profile presented in Figure 8.

### Example 2. (Comparative Example)

Starting material, which is tadalafil in the mixture of acetone and isopropanol (API-in-solution) is combined with carrier (excipients) in the process of wet granulation, i.e. high shear granulation. Obtained blend is dried in temperature of 75 °C, sieved and mixed with other excipients. Finally the blend is mixed with lubricant, then compressed to form tablets. Final composition is given in Example 1 Table 2.

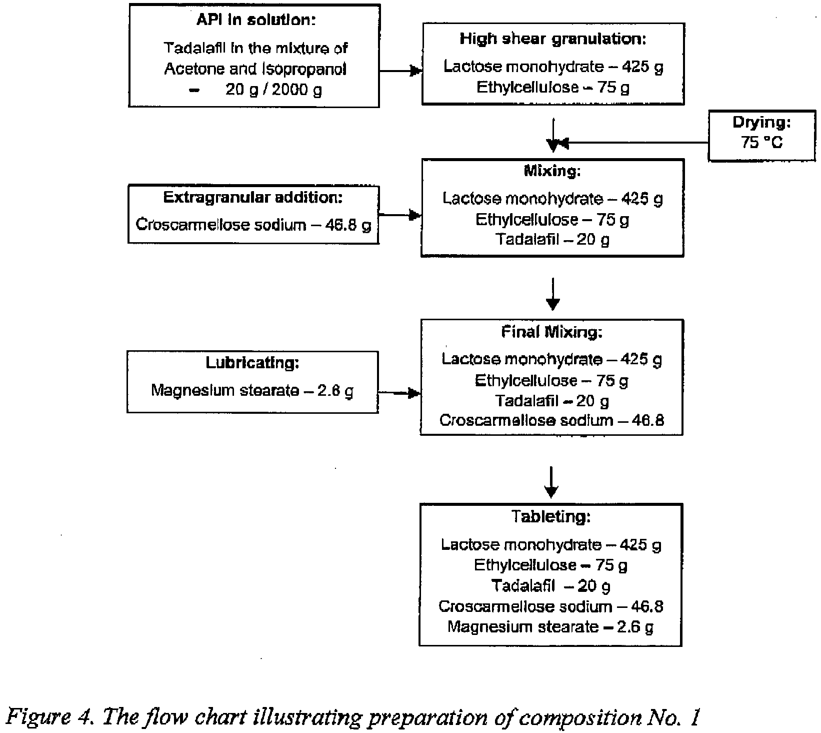

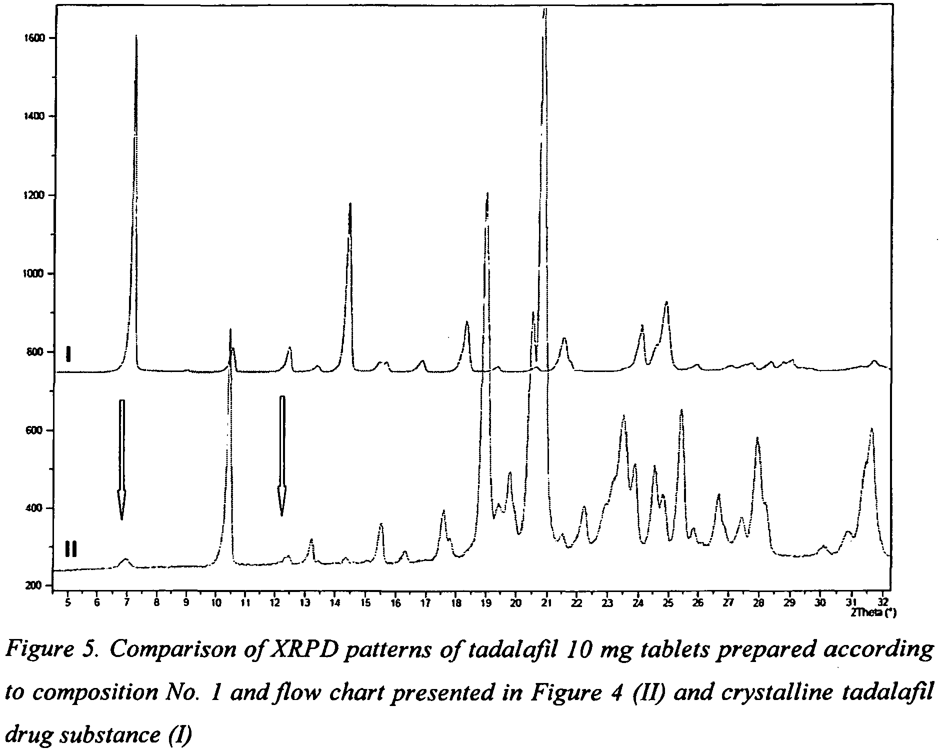

XRPD pattern of tadalafil 10 mg tablets prepared according to composition No. 1 (Table 2) and flow chart presented in Figure 4, shows appearance peaks which are characteristic to crystalline tadalafil (marked with arrows). Technological process presented in Figure 4 do not gives as effect specific structure comprising tadalafil incorporated in superficial layer of ethylcellulose. Moreover this technological process do not result in improvement of dissolution rate (Figure 7).

### Example 3.

API-in-solution can be introduced into technological process as a mixture with one or more other substances, which can improve its physical and chemical properties, but do not negatively influence its pharmaceutical availability determined as rate of dissolution.

To starting material, which is tadalafil in the mixture of acetone and isopropanol (API-in-solution), other substance, e.g. polyoxyethylene sorbitan fatty acid esters, can be introduce. This solution is next combined with carrier (excipients) in the process of hot melt granulation. Obtained blend is mixed with other excipients and finally with lubricant, then compressed to form tablets.

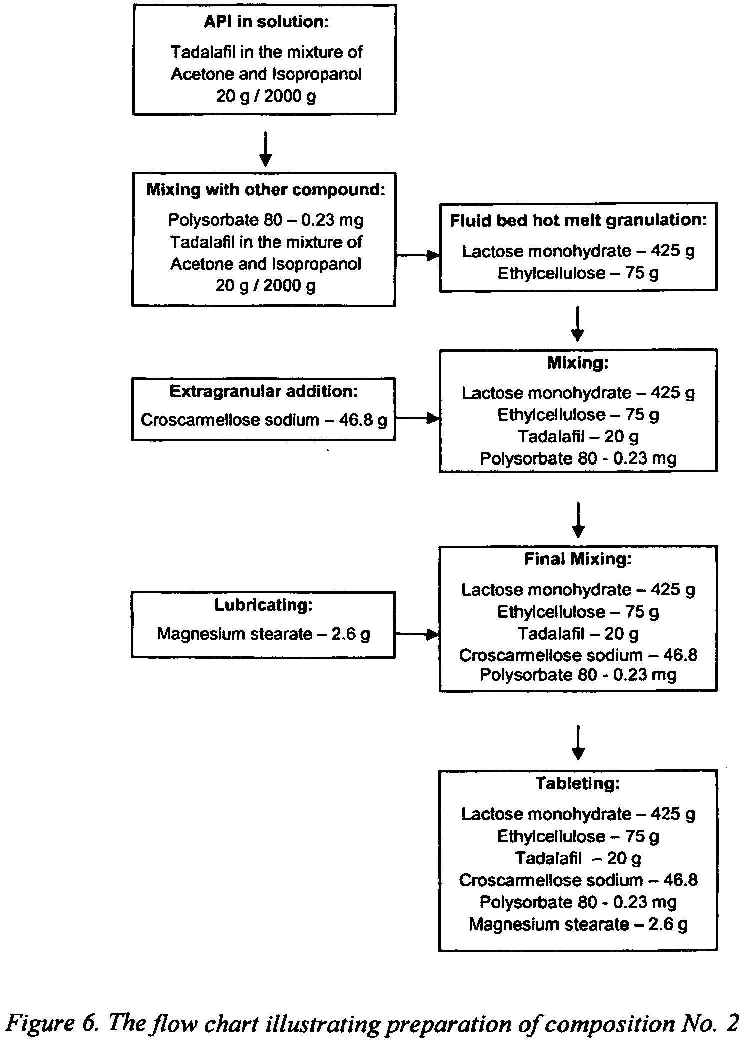

**Table 3. Tadalafil tablets 10 mg - composition No. 2**

| Substance name | amount [mg/tabl] | amount [%] |
|---|---|---|
| Tadalafil | 10.000 | 3.80 |
| Tween | 0.208 | 0.08 |
| Laktose monohydrate | 212.200 | 81.62 |
| Ethylocellulose | 13.000 | 5.0 |
| Croscarmellose sodium | 23.400 | 9.0 |
| Magnesium stearate | 1.300 | 0.5 |
| Total: | 260.108 | 100.00 |

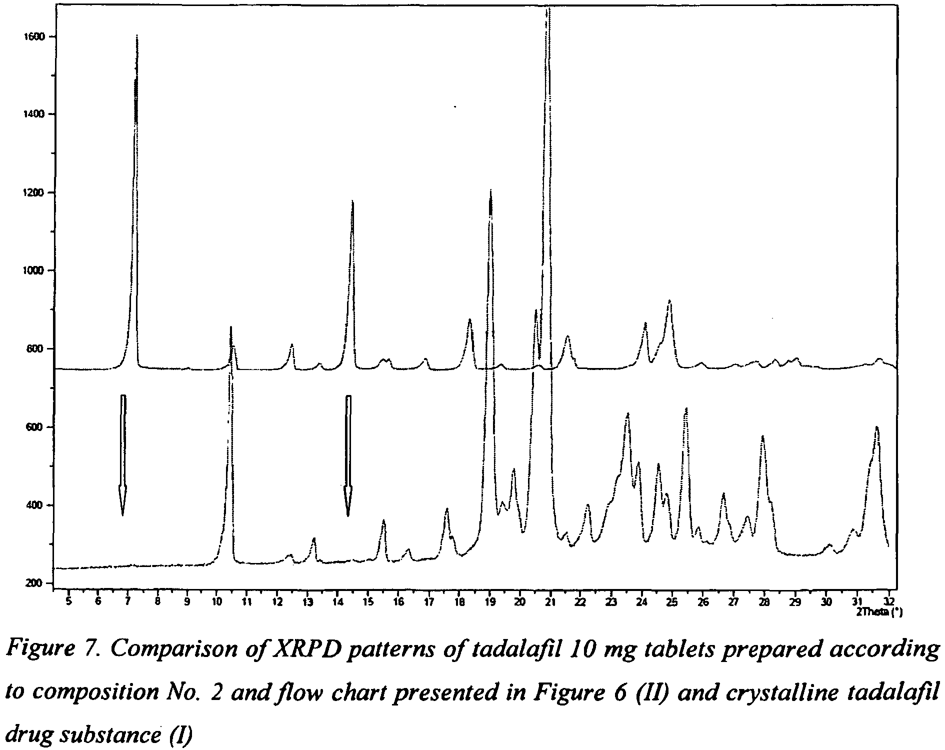

XRPD pattern of tadalafil 10 mg tablets prepared according to composition No. 2 (Table 3) and flow chart presented in Figure 6, shows lack of the most intensive peaks characteristic to crystalline tadalafil (marked with arrows). Moreover it is characterized by improved solubility of tadalafil, in similar way as is presented in example 1.

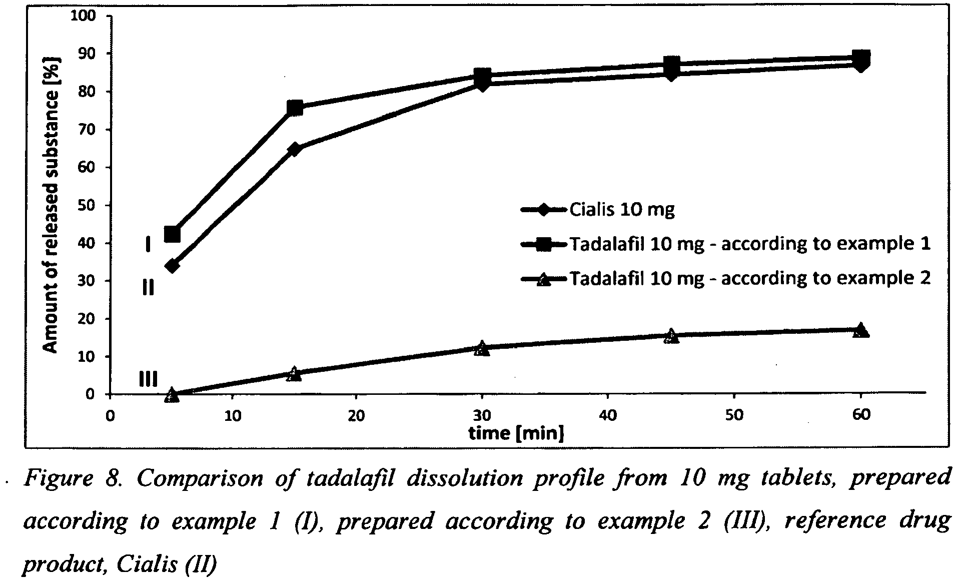

## Claims

1. Process for the production of pharmaceutical composition comprising the steps of
a. preparing a solution of tadalafil in organic solvent,
b. carrying up tadalafil onto a water-insoluble polymer mixed with a diluent by fluid-bed hot-melt granulation, where the water-insoluble polymer is ethylcellulose,
c. mixing the granulation with one or more further excipients.

2. Process according to claim 1 **characterized by** that the organic solvent is selected from the group of methanol, ethanol, isopropanol, acetone and a mixture thereof.

3. Process according to any of claims 1 and 2 **characterized by** that the that the organic solvent is the mixture of aceton and isopropanol.

4. Process according to any of claims 1 to 3 **characterized by** that the fluid-bed granulation is carried out at the temperature of 50-250 °C.

## Patentansprüche

1. Verfahren für die Herstellung einer pharmazeutischen Zusammensetzung, welches Verfahren die Schritte umfasst:
a. Herstellen einer Auflösung von Tadalafil in einem organischem Lösemittel;
b Auftragen von Tadalafil auf einem wasserunlöslichen Polymer gemischt mit einem Streckmittel mit Hilfe einer Fließbett-Heißschmelz-Granulierung, wobei das wasserunlösliche Polymer Ethylcellulose ist;
c. Mischen der Granulierung mit einem oder mehreren weiteren Arzneimittelträgern.

2. Verfahren nach Anspruch 1, **dadurch gekennzeichnet, dass** das organische Lösemittel ausgewählt ist aus der Gruppe bestehend aus Methanol, Ethanol, Isopropanol, Aceton und einer Mischung davon.

3. Verfahren nach einem der Ansprüche 1 und 2, **dadurch gekennzeichnet, dass** das organische Lösemittel die Mischung von Aceton und Isopropanol ist.

4. Verfahren nach einem der Ansprüche 1 bis 3, **dadurch gekennzeichnet, dass** die Fließbett-Granulierung bei einer Temperatur von 50° bis 250°C ausgeführt wird.

## Revendications

1. Procédé pour la production d'une composition pharmaceutique comprenant les étapes de :
a. préparer une solution de tadalafil en solvant organique ;
b. transporter le tadalafil sur un polymère insoluble dans l'eau mélangé avec un diluant par granulation par fusion en lit fluidisé, où le polymère insoluble dans l'eau est l'éthylcellulose,
c. mélanger la granulation avec un ou plusieurs autres excipients.

2. Procédé selon la revendication 1, **caractérisé par le fait que** le solvant organique est choisi dans le groupe de méthanol, éthanol, isopropanol, acétone et un mélange de ceux-ci.

3. Procédé selon l'une quelconque des revendications 1 et 2, **caractérisé par le fait que** le solvant organique est le mélange d'acétone et d'isopropanol.

4. Procédé selon l'une quelconque des revendications 1 à 3, **caractérisé par le fait que** la granulation en lit fluidisé est effectuée à la température de 50-250°C.
